# EUROPEAN PATENT APPLICATION

(11) **EP 4 644 559 A1**
(43) Date of publication of application: **05.11.2025**
(21) Application number: 24173609.9
(22) Date of filing: 30.04.2024
(51) Int. Cl.: C12P 7/40, C12N 9/02, C12N 11/14

(54) **METHOD FOR ENZYME-ASSISTED PLASMA CHEMICAL REACTION**

(71) Applicant: Leibniz-Institut für Plasmaforschung und Technologie e.V., 17489 Greifswald (DE)
(72) Inventor: BORGHEI, Sepideh, 17489 Greifswald (DE); KOLB, Jürgen, 17489 Greifswald (DE); BRÜSER, Volker, 17489 Greifswald (DE); HAHN, Veronika, 17489 Greifswald (DE)
(74) Representative: Schulz Junghans Patentanwälte PartGmbB

(57) **Abstract**

The present invention relates to a method for performing an enzymatic reaction, wherein the enzymatic reaction is performed in a reaction volume in the presence of water using plasma-generated species. The method is characterized in that plasma is generated in said reaction volume.

## Description

### Field

The present invention relates to a method for performing an enzymatic reaction using plasma-generated species. Plasma generation and enzymatic reaction are performed in the same reaction volume.

### Background

Physical plasma has generated significant attention in the field of chemical processing due to its unique properties. The plasma is often investigated together with catalysts to enhance the conversion of specific compounds.

WO2020/007576A1 is directed towards enzyme catalysed reactions using enzymes such as unspecific peroxygenase and horseradish peroxidase in combination with plasma treatment to generate the substrate H₂O₂. The enzymes comprise a prosthetic heme group that acts as co-factor/cofactor for the conversion of an organic compound to the desired product. Product yields were investigated after direct exposure of the dissolved enzyme to the plasma but found low compared to a spatially separated approach of first treating a buffer with plasma and then adding the buffer to another vial containing the enzyme and organic compound.

A. Yayci et al. (Febr. 2020) observed that direct exposure of enzymes to plasma treatment could lead to reduced enzyme activity, potentially due to factors such as enzyme inactivation, substrate limitation, or degradation of the product by short-lived plasma species.

In the study on Microscale Atmospheric Pressure Plasma Jet as a Source for Plasma-Driven Biocatalysis presented by A. Yayci et al. (Aug. 2020), plasma and enzyme were spatially separated. A microscale atmospheric pressure plasma jet (µAPPJ) was used to treat a liquid buffer surface. The resulting products were then transferred to the immobilized enzymes by stirring the liquid in a rotating bed reactor, the plasma-treated buffer was used to supply hydrogen peroxide for the biocatalytic reactions. The enzyme, a recombinant unspecific peroxygenase from *Agrocybe aegerita* (rAaeUPO), was used as a biocatalyst and was added to the reaction solution after plasma treatment. The enzyme was either added directly to the plasma-treated buffer or immobilized on support beads and placed in a rotating bed reactor for continuous catalytic reactions.

The present invention aims to facilitate plasma-driven biocatalysis by a one-pot approach that combines plasma generation and enzymatic reaction within one reaction volume at the same time. Furthermore, particularly short-living plasma generated species allow for performing an enzymatic reaction without the need of adding an otherwise required cofactor.

Based on the above-mentioned state of the art, the objective of the present invention is to provide means and methods to facilitate plasma-driven biocatalysis. This objective is attained by the subject-matter of the independent claims of the present specification, with further advantageous embodiments described in the dependent claims, examples, figures and general description of this specification.

### Summary of the Invention

A first aspect of the invention relates to a method for performing an enzymatic reaction, wherein the enzymatic reaction is performed in the presence of water in a reaction vessel, and the enzymatic reaction is supported by generating a plasma in said reaction vessel.

### Terms and definitions

### General

For purposes of interpreting this specification, the following definitions will apply and whenever appropriate, terms used in the singular will also include the plural and vice versa. In the event that any definition set forth below conflicts with any document incorporated herein by reference, the definition set forth shall control.

The terms "comprising", "having", "containing", and "including", and other similar forms, and grammatical equivalents thereof, as used herein, are intended to be equivalent in meaning and to be open-ended in that an item or items following any one of these words is not meant to be an exhaustive listing of such item or items, or meant to be limited to only the listed item or items. For example, an article "comprising" components A, B, and C can consist of (i.e., contain only) components A, B, and C, or can contain not only components A, B, and C but also one or more other components. As such, it is intended and understood that "comprises" and similar forms thereof, and grammatical equivalents thereof, include disclosure of embodiments of "consisting essentially of" or "consisting of."

Where a range of values is provided, it is understood that each intervening value, to the tenth of the unit of the lower limit, unless the context clearly dictates otherwise, between the upper and lower limit of that range and any other stated or intervening value in that stated range, is encompassed within the disclosure, subject to any specifically excluded limit in the stated range. Where the stated range includes one or both of the limits, ranges excluding either or both of those included limits are also included in the disclosure.

Reference to "about" a value or parameter herein includes (and describes) variations that are directed to that value or parameter per se. For example, description referring to "about X" includes description of "X."

As used herein, including in the appended claims, the singular forms "a", "or" and "the" include plural referents unless the context clearly dictates otherwise.

"And/or" where used herein is to be taken as specific recitation of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

Unless defined otherwise, all technical and scientific terms used herein have the same meaning as commonly understood by one of ordinary skill in the art (e.g., in cell culture, molecular genetics, nucleic acid chemistry, hybridization techniques and biochemistry, organic synthesis). Standard techniques are used for molecular, genetic, and biochemical methods (see generally, Sambrook et al., Molecular Cloning: A Laboratory Manual, 4th ed. (2012) Cold Spring Harbor Laboratory Press, Cold Spring Harbor, N.Y. and Ausubel et al., Short Protocols in Molecular Biology (2002) 5th Ed, John Wiley & Sons, Inc.) and chemical methods.

The term *oxidoreductase* in the context of the present specification relates to an enzyme falling into class EC (Enzyme Commission) 1 of the EC number classification of enzymes, particularly EC 1.17, EC 1.2, EC 1.4, EC 1.5 and EC 1.1. Oxidoreductases catalyse the transfer of electrons from one molecule to another.

The term *ligase* in the context of the present specification relates to an enzyme falling into class EC 6 of the EC number classification of enzymes, particularly EC 6.3. Ligases join together two molecules by synthesis of new C-O, C-S, C-N or C-C bonds with simultaneous breakdown of ATP.

The term *formate dehydrogenase* in the context of the present specification relates to an enzyme classified by EC (Enzyme Commission) numbers EC 1.17.1.9 (NADH-dependent) and EC 1.2.2.1 (cytochrome-dependent), particularly EC 1.17.1.9. A non-limiting example for a suitable formate dehydrogenase is that from *Candida boidinii* (UniProt accession number 013437).

The term *amine dehydrogenase* in the context of the present specification relates to an enzyme classified by EC (Enzyme Commission) number 1.4.99.3. Particularly, the amine dehydrogenase is tryptophan tryptophylquinone-dependent (TTQ-dependent).

The term *glutamine synthetase* in the context of the present specification relates to an enzyme classified by EC (Enzyme Commission) number 6.3.1.2. Particularly, the glutamine synthetase is ATP-dependent.

The term *imine reductase* in the context of the present specification relates to an enzyme classified by EC (Enzyme Commission) number 1.5.1.48. Particularly, the imine reductase is NAD(P)H-dependent.

The term ene reductase in the context of the present specification relates to an enzyme classified by EC (Enzyme Commission) number 1.6.99.1.

The term *nitroreductase* in the context of the present specification relates to an enzyme classified by EC (Enzyme Commission) number 1.5.1.34.

The term *aldo-keto reductase* protein superfamily comprise different NAD(P)H-dependent oxidoreductases. *Ketone reductases (KREDs)* belong to this family and are classified by EC (Enzyme Commission) number 1.1.1.

The term *alcohol dehydrogenase* in the context of the present specification relates to an enzyme classified by EC (Enzyme Commission) number 1.1.1.1. Particularly, the alcohol dehydrogenase is NADH-dependent.

The term *aldose reductase or aldehyde reductase* in the context of the present specification relates to an enzyme classified by EC (Enzyme Commission) number 1.1.1.21. Particularly, the aldose reductase is NADPH-dependent.

The term *cofactor-dependent enzyme* in the context of the present specification relates to an enzyme that usually catalyses a chemical reaction by utilizing a cofactor, i.e. a coenzyme/cosubstrate or prosthetic group, particularly a coenzyme. The term relates in particularly to an enzyme that usually catalyses a chemical reaction by utilizing
a coenzyme/cosubstrate selected from
   - nicotinamide adenine dinucleotide in oxidized (NAD⁺) and reduced form (NADH),
   - nicotinamide adenine dinucleotide phosphate in oxidized (NADP⁺) and reduced form (NADPH),
   - flavine adenine dinucleotide in oxidized (quinone form/FAD) and reduced form (hydroquinone form/FADH₂),
   - adenosine triphosphate (ATP) and adenosine diphosphate (ADP),
   - S-adenosyl methionine (SAM) and S-adenosyl homocysteine (SAH),
   - Pyridoxal phosphate (PLP),
or a prosthetic group selected from
   heme
   cytochrome P450.

When performing the inventive method, the cofactor-dependent enzyme is particularly used without its respective co-factor.

Any patent document cited herein shall be deemed incorporated by reference herein in its entirety.

### Detailed Description of the Invention

A first aspect of the invention relates to a method for performing an enzymatic reaction, wherein the enzymatic reaction is performed in the presence of water in a reaction vessel, and the enzymatic reaction is supported by generating a plasma in said reaction vessel.

In an alternative embodiment, the invention relates to a method for performing an enzymatic reaction, wherein the method comprises
- adding water and an enzyme to a reaction vessel,
- generating plasma in said reaction vessel.

There is increasing demand for alternative reaction pathways to mitigate issues such as global warming and climate change, environmental pollution including the contamination of water, or exploitation of natural resources. For instance, the catalytic conversion of CO₂ to biofuels and fine chemicals may contribute to the development of a sustainable low-carbon economy with reduced emission of greenhouse gases, enzymatic waste water treatment to remove toxic substances may provide clean water and the degradation or modification of substances such as polymers may led to a more sustainable pathway towards valuable compounds. Enzymatic reactions are of high interest in this context. The present invention aims to facilitate biocatalysis by providing means and methods to perform an enzymatic reaction using plasma-generated species.

In the combined process (simultaneous enzymatic reaction and plasma generation), the equilibrium of the enzyme reactions can be shifted by plasma. Additionally, the selectivity of plasma chemical reaction can be controlled by enzyme type. Moreover, special enzymes need a coenzyme/cofactor. However, for these enzymes no addition of the coenzyme/cofactor is needed when plasma is used.

Instead of metal or ceramic catalysts, the invention is based on the combination with enzymes, which are known to catalyse specific reactions in biological cells. Therefore, the invention relates to methods of enzymatic catalysed plasma chemical conversion in the presence of water. Plasma process and enzymatic reaction occurs in the same volume of process.

Possible applications for the inventive methods are enzymatic waste water treatment to remove toxic substances (clean water), the improved production of biofuels (clean energy), the degradation or modification of substances such as polymers (e.g. plastic, lignin) towards valuable compounds, greener synthesis of fine chemicals consuming fewer resources and generating less waste (responsible consumption and production), and energy efficient CO₂ conversion to reduce greenhouse gases (climate action).

Particularly short-living plasma-generated species may fulfill the function of an otherwise required co-factor. Thus, it is beneficial if the plasma is generated in the same reaction vessel in which the enzyme is present. Subsequently, plasma generation and enzymatic reaction occur at the same time.

In certain embodiments, the enzymatic reaction and plasma generation occur simultaneously.

The inventive method may be applied for the reduction of CO₂ with H₂ to formate (the conjugate base of formic acid) by using the enzyme formate dehydrogenase in combination with plasma-generated species. As described above, the use of plasma may replace the use of a co-factor. In this particular application, the presence of the co-factor NADH was not necessary as an electron donor for the enzyme reaction.

In certain embodiments, the enzymatic reaction is performed in the absence of a co-factor.

The inventive method was developed for the reduction of CO₂ with H₂ to formate (the conjugate base of formic acid) by the enzyme formate dehydrogenase but is in principle not limited to this particular enzyme or the respective conversion. Regardless, the capture of CO₂ into the energy carrier formic acid is an application of significant relevance by itself.

For instance, the inventive method is also applicable to the formation of amines using amine dehydrogenases, amino acid dehydrogenases, glutamine synthetase, imine reductase, the degradation of alcohols using an alcohol dehydrogenase, the catalysis of redox reactions using oxidoreductases, the reduction of aldehydes and ketones using aldehyde reductases and aldose reductase, respectively, or the reduction of nitrogen-containing compounds with nitroreductases.

In certain embodiments, the enzyme that catalyses said enzymatic reaction is a cofactor-dependent enzyme.

In certain embodiments, the enzyme that catalyses said enzymatic reaction is an oxidoreductase or ligase.

In certain embodiments, the enzyme that catalyses said enzymatic reaction is an oxidoreductase.

In certain embodiments, the oxidoreductase is selected from an NAD(P)H-dependent oxidoreductase, a cytochrome-dependent oxidoreductase, tryptophan tryptophylquinone-dependent oxidoreductase, ATP-dependent ligase.

In certain embodiments, the enzyme that catalyses said enzymatic reaction is selected from a formate dehydrogenase, an amine dehydrogenase, a glutamine synthetase, an imine reductase, an alcohol dehydrogenase, an aldehyde reductase, an aldose reductase, a nitroreductase.

In certain embodiments, the enzyme that catalyses said enzymatic reaction is a NADH-dependent formate dehydrogenase.

To prevent enzyme destruction when in contact with plasma, the enzyme is immobilized on a porous solid support. Either adsorption or covalent bonding for enzyme immobilization have been tested. The findings indicated that the covalent immobilization is more efficient, allowing the enzymes to remain active.

In certain embodiments, the enzyme that catalyzes said enzymatic reaction is immobilized on a carrier.

In certain embodiments, the enzymatic reaction is selected from an oxidation/reduction reaction.

In certain embodiments, the enzyme that catalyzes said enzymatic reaction is immobilized.

In certain embodiments, the enzyme that catalyzes said enzymatic reaction is immobilized on a carrier.

In certain embodiments, the carrier is porous.

In certain embodiments, the pore volume is 66 - 75%. The pores vary in dimension. They are usually bigger on the outside and more narrow towards the center. Average diameter of the pores is around 50 nanometers.

In certain embodiments, the carrier is selected from a glass bead, a ceramic bead or ceramic granulate, a plastic bead or plastic fabric. In certain embodiments, the plastic bead or plastic fabric is made of polyacrylic, styrene, methacrylate or polystyrene.

In certain embodiments, the carrier comprises a functional group selected from epoxide, alkyl, phenyl, octadecyl, sulphonic, tertiary amine, quaternary ammonium.

In certain embodiments, the enzyme is immobilized on a carrier selected from a glass bead, ceramic beads or granulate, plastic beads or fabric (polyacrylic, styrene, methacrylate, polystyrene), with functional groups (epoxide, alkyl, phenyl, octadecyl, sulphonic, sulphonic, tertiary amine, quaternary ammonium type).

In certain embodiments, the enzyme is immobilized by adsorption (polar or apolar), cationic, anionic or covalent bonding (polar or apolar), particularly by covalent bonding.

In certain embodiments, the reaction vessel further contains one or more reactants for the enzymatic reaction selected from CO₂ and H₂. For instance, for the reduction of CO₂ with H₂ to formate using a formate dehydrogenase, both CO₂ and H₂ are added to the reaction vessel.

In certain embodiments, the reaction vessel further contains a reactant for the enzymatic reaction selected from CO₂ and H₂.

In certain embodiments, the ratio of said starting material and said substrate is 10:1 to 1:10, particularly 1:1 to 1:3, more particularly 1:3.

In certain embodiments, the ratio of H₂ to CO₂ is 10:1 to 1:10, particularly 1:1 to 1:3, more particularly 1:3.

In certain embodiments, the enzymatic reaction is performed in the presence of Ar, particularly 10 to 90 % Ar, more particularly 20% Ar. Argon reduces the ignition voltage and makes the plasma more stable.

In certain embodiments, the plasma is generated by a discharge selected from
- dielectric barrier discharge (DBD),
- corona discharge,
- microwave discharge,
- spark discharge,
- inductively-limited spark discharge
- rf plasma jet,
- dc plasma jet
- gliding arc, or
- microwave plasma jet.

In certain embodiments, the plasma is generated by a discharge selected from dielectric barrier discharge (DBD.

When performing the method of the invention, the temperature in the reaction vessel is adjusted in such a way that the enzyme is active.

Wherever alternatives for single separable features are laid out herein as "embodiments", it is to be understood that such alternatives may be combined freely to form discrete embodiments of the invention disclosed herein.

The invention is further illustrated by the following examples and figures, from which further embodiments and advantages can be drawn. These examples are meant to illustrate the invention but not to limit its scope.

### Description of the Figures

- Fig. 1: shows the experimental set-up
- Fig. 2: shows the formate concentration mg/L with respect to H₂:CO₂ ratio in the mixture for a water flow of 1.4 ml/min. Three independent experiments (COV-1+enz,1; COV-1+enz,2; COV-1+enz,3) show significantly higher concentrations compared to an operation of the packed-bed reactor without any enzymes. COV-1,1; COV-1,2; COV-1+enz, 1; COV-2+enz3; ADS-1+enz,1; ADS-1+enz,2; water: deionized water; control: the combination of water, DBD reactor filed with immobeads 150-600 µm) and glassbeads (1 mm) but no enyzme, and CO2, H2 and Ar without plasma treatment

### Explanations:

COV-1,1: mixture glass beads (1 mm) and immobeads (apolar type IB-COV-1, 150-600 µm), without enzyme
COV-1,2: repetition of COV-1,1
COV-1+enz, 1: mixture glass beads (1 mm) and immobeads (apolar type IB-COV-1, 150-600 µm), with enzyme with covalent bonding method
COV-2+enz2 and 3: repetition of COV-1+enz, 1
ADS-1+enz,1: mixture glass beads (1 mm) and immobeads (apolar type IB-ADS-1 (alkyl, 300-700 µm), with enzyme with adsorption bonding method
ADS-1+enz,2: repletion of ADS-1+enz,1

All the experiments were carried out with deionized water

### Examples

The experiments were conducted using different ratios H₂:CO₂, namely 1:1 and 1:3, both with and without the presence of 20% Ar, within a coaxial dielectric barrier discharge powered by positive nano-second pulsed high-voltage discharges. The reactor volume was filled with a mixture of immobilized formate dehydrogenase as enzyme and glass beads (1 mm). Remarkably, the inclusion of immobilized enzymes led to a substantial increase from 0.07 to 0.73 mg/L in formate formation in the liquid. This enhancement was observed compared to samples containing only glass beads and deionized water, without any immobilized enzymes in the reactor. Usually, enzymes require co-factors, which could be replaced by reactive species provided by the plasma. In this particular application, the presence of NADH was not necessary as an electron donor for the enzyme reaction.

### Example 1: Plasma-assisted formic acid formation by formate dehydrogenase in the absence of a co-factor

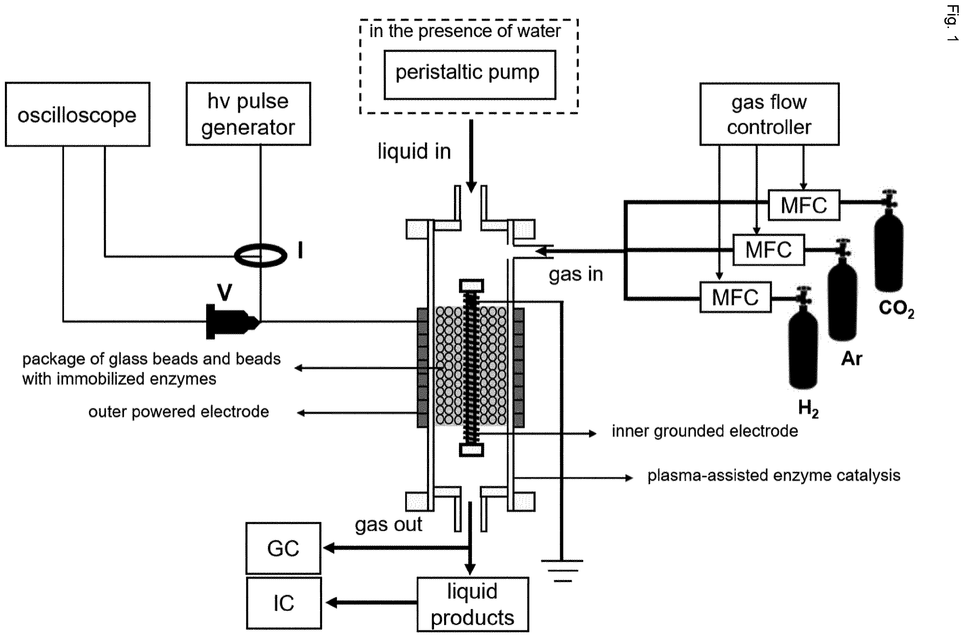

The immobilized enzyme formate dehydrogenase has been tested in an enzyme-assisted plasma reactor (Figure 1) to produce formic acid and different parameters including different mixture ratios of H₂:CO₂, two different immobilization methods including adsorption or covalent bonding have been tested in the laboratory scale.

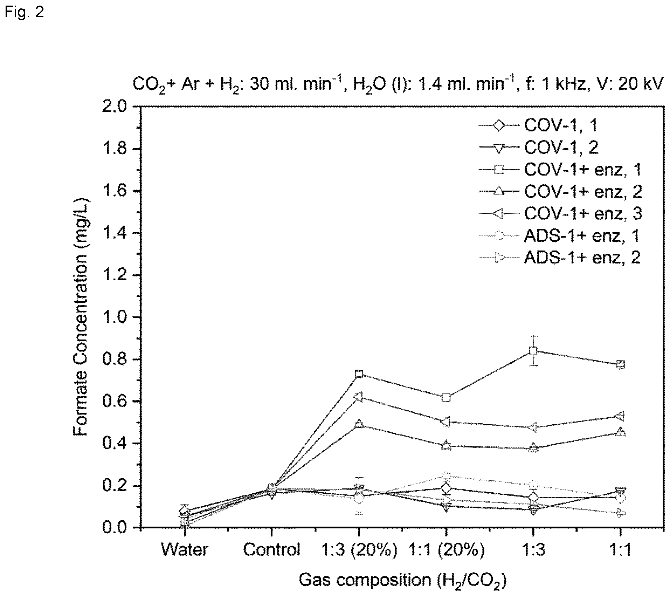

The inclusion of immobilized enzymes led to a substantial increase from 0.07 to 0.73 mg/L in formate formation in the liquid samples (the treated deionised water, which is extracted from the reactor after plasma treatment. - Figure 2). 0.73 mg/L is an average for the test 1:3, 20% of Ar for COV-1-1+enz1 derived from three experiments. Increasing the concentration of enzyme have been also tested. Obtained results indicated that the rise in the concentration of enzyme in the solution from 90 mg to 360 mg led to an increase in the formation of formate by the factor of approximately 3 (not shown in the figures).

Furthermore, to exclude an influence of a NADH-containing solution used for enzyme immobilization, a buffer solution without any NADH (coenzyme/cofactor) was used, interestingly, enzyme was still active and formate was formed in the presence of plasma, although in a lower concentration compared to the experiment with NADH solution, meaning that in the presence of plasma the enzyme does not need any additional coenzyme/cofactor. The formate concentration with buffer solution in 90 mg of the enzyme per 12 ml of buffer solution was 0.3864 mg/L.

Notably, experiments were carried out three times to ensure reproducibility.

### Experimental setup

The experimental setup used in this study is illustrated in Figure 1. A borosilicate glass tube with inner and outer diameters of 17 mm and 22 mm, respectively, served as the dielectric barrier in an asymmetric dielectric barrier discharge (DBD) plasma reactor. The dielectric barrier had a wall thickness of 2.5 mm and a length of 130 mm, enclosing a stainless-steel threaded rod (M12) at the central axis, which acted as the grounded electrode. This arrangement resulted in a discharge gap of 2.5 mm. The dielectric was tightly wrapped with nine aluminum rings, each 10 mm in height and 32 mm in outer diameter, totaling a discharge length of 90 mm. The reactor was filled with the mixture of glass beads (1mm in diameter) and beads with immobilized enzymes (immobeads, ChiralVision B.V., Den Hoorn, Netherlands; 150-600 µm or 300-700 µm in diameter). The glass beads provide more discharge gaps in the reactor yielding to a stronger discharge. The use of immobeads alone would hinder the ignition due to the package density of the immobeads. The glass beads provide more space between the beads due to the bigger diameter compared to immobeads.

The reactor setup involved the controlled introduction of a gas mixture consisting of CO₂, H₂, and Ar, regulated by individual mass flow controllers (MKS Instruments, 647C). The gases were introduced into the reactor at a constant flow rate of 30 ml/min, with entry from the right side and expulsion of gaseous products from the bottom. Concurrently, deionized water, regulated by a peristaltic pump operating at a fixed flow rate of 1.4 ml/min, was directed into the reactor from the top. Liquid products were collected in a beaker positioned at the bottom of the reactor. Two different gas ratios were examined during the experiment. Initially, with a fixed Ar concentration of 20%, the H₂:CO₂ ratio was set at 1:1 and 1:3, respectively. Subsequently, the experiments were repeated without the presence of Ar.

Gas chromatography (GC) was employed for the analysis of gaseous products, while ion chromatography (IC) was utilized for the analysis of liquid products including formate.

Moreover, the discharge was powered by a positive high-voltage ns-pulsed power supply operating at a fixed repetition rate of 1 kHz. The voltage amplitude and pulse duration were set at 20 kV and 500 ns, respectively.

### Enzyme immobilization

The immobilized enzyme formate dehydrogenase (UniProt accession number 013437) has been tested for this invention in an enzyme-equipped plasma reactor (Figure 1) to produce formic acid. Two different immobilization methods including adsorption or covalent attachment have been tested in the laboratory scale.

The beads used in this study did not require prewashing. To ensure optimal enzyme binding, the material's pH was adjusted accordingly. Initially, 25 ml of deionized water was added for each 4 grams of the beads (wet weight), followed by thorough stirring. Subsequently, the pH was adjusted to the desired range of 6.5-7.5 using sodium hydroxide solution. Following pH adjustment, the beads were washed with three bed volumes of deionized water, and any excess water was removed. Once completed, the beads were ready for use.

For enzyme immobilization, two types of immobeads were utilized: for covalent attachment an apolar type IB-COV-1 (epoxide, butyl, 150-600 µm) and for adsorption an apolar type IB-ADS-1 (alkyl, 300-700 µm). The immobeads were purchased from Chiral Vision.

Product: IB-COV-1; Type: Covalent, apolar; Matrix: Polyacrylic; Functional group: epoxide, butyl; Particle size (µm) 150-600; Water 75 ml.

Product: IB-ADS-1; Type: Adsorption, apolar; Matrix: Polyacrylic; Functional group: alkyl; Particle size (µm) 300-700; Water 71 ml.

A proportion of up to 12 ml of enzyme solution (containing 90 mg formate dehydrogenase and 12 ml NADH (β-Nicotinamide Adenine Dinucleotide; 49.75 mg NADH/ 50 ml sodium phosphate buffer solution) per 3 grams of immobeads (dry weight) was employed at 4°C. The pH of the solution was adjusted to approximate the optimal pH range for the enzyme (around 7-7.5). This immobilization process was replicated using 24 ml of enzyme solution (containing 180 mg formate dehydrogenase and 24 ml NADH), as well as 48 ml of enzyme solution (containing 360 mg formate dehydrogenase and 48 ml NADH) for three grams of immobeads. Furthermore, these conditions were reassessed by substituting the NADH solution with sodium phosphate buffer solution.

For screening purposes, disposable 1.5 ml vials containing 200 mg of beads and 800 µl of enzyme solution were found to be convenient. The beads and enzyme were mixed on ice at 4°C for 2 minutes and then left overnight in a refrigerator at 4°C without agitation. After overnight incubation, the supernatant was checked to assess the degree of enzyme binding. The beads were then washed 5 times with two bed volumes of cold deionized water to remove any unbound enzyme.

### Enzymatic assay of formate dehydrogenase

The enzymatic assay of formate dehydrogenase involves the conversion of formate + β-NAD⁺ (β-Nicotinamide Adenine Dinucleotide, Oxidized Form) → CO₂ + β-NADH (β-Nicotinamide Adenine Dinucleotide, Reduced Form). In this assay, the temperature (T) and pH were set to 37°C and 7.0, respectively. Spectrophotometric absorbance measurement was fixed at 340 nm in a light path of 1 cm. The following reagents were used for this assay: A) 200 mM (399.6 mg/100 ml deionized water) of sodium phosphate buffer with pH 7.0 set at 37°C. B) 200 mM (136.02 mg/ml deionized water) of sodium formate solution. C) 10.5 mM (20.898 mg/3 ml deionized water) of β-Nicotinamide Adenine Dinucleotide solution (β-NAD). D) 1.5 mM (49.75 mg/ 50 ml deionized water; reagent A) of β-Nicotinamide Adenine Dinucleotide Solution (Enzyme Diluent). E) Formate dehydrogenase enzyme solution consisting of lyophilized enzyme (manufacturer, town, country) and reagent D.

The specified volumes of reagents were pipetted into suitable cuvettes for the test and blank samples. The components were then mixed by inversion and allowed to equilibrate to 37°C. The absorbance at 340 nm was monitored until it reached a constant value using a spectrophotometer which was connected to a thermostat. Finally, the rate of absorbance change (ΔA340nm/minute) was calculated using the maximum linear rate observed for both the test and blank samples.

### Cited references:

WO2020/007576A1
A. Yayci et al. (Febr. 2020) Plasma-Driven in Situ Production of Hydrogen Peroxide for Biocatalysis. ChemSusChem 13:2072-2079
A. Yayci et al. (Aug. 2020) Microscale Atmospheric Pressure Plasma Jet as a Source for Plasma-Driven Biocatalysis. ChemCatChem 12:5893-5897

All scientific publications and patent documents cited in the present specification are incorporated by reference herein.

## Claims

1. A method for performing an enzymatic reaction, wherein the enzymatic reaction is performed in the presence of water in a reaction vessel, and
the enzymatic reaction is supported by generating a plasma in said reaction vessel.

2. The method according to claim 1, wherein the enzymatic reaction is performed in the absence of a co-factor.

3. The method according to any of the preceding claims, wherein the enzyme that catalyses said enzymatic reaction is a cofactor-dependent enzyme, particularly an oxidoreductase, ligase.

4. The method according to claim 3, wherein
- the oxidoreductase is selected from an NAD(P)H-dependent oxidoreductase, a cytochrome-dependent oxidoreductase, tryptophan tryptophylquinone-dependent oxidoreductase,
- ATP-dependent ligase.

5. The method according to any of the preceding claims, wherein the enzyme that catalyses said enzymatic reaction is selected from a formate dehydrogenase, an amine dehydrogenase, a glutamine synthetase, an imine reductase, an alcohol dehydrogenase, an aldehyde reductase, an aldose reductase, a nitroreductase.

6. The method according to any of the preceding claims, wherein the enzyme that catalyses said enzymatic reaction is a NADH-dependent formate dehydrogenase.

7. The method according to any of the preceding claims, wherein the enzyme that catalyzes said enzymatic reaction is immobilized on a carrier.

8. The method according to claim 7, wherein the carrier is porous comprising at least one open pore.

9. The method according to any of claims 7 or 8, wherein the carrier is selected from a glass bead, a ceramic bead or ceramic granulate, a plastic bead or plastic fabric.

10. The method according to any of claims 7 to 8, wherein the carrier comprises a functional group selected from epoxide, alkyl, phenyl, octadecyl, sulphonic, tertiary amine, quaternary ammonium.

11. The method according to any of claims 8 to 10, wherein the enzyme is immobilized within said open pore.

12. The method according to any of claims 7 to 11, wherein the enzyme is immobilized by adsorption (polar or apolar), cationic, anionic or covalent bonding (polar or apolar), particularly by covalent bonding.

13. The method according to any of the preceding claims, wherein said reaction vessel further contains one or more reactants for the enzymatic reaction selected from CO₂ and H₂.

14. The method according to claim 13, wherein the ratio of H₂ to CO₂ is 10:1 to 1:10, particularly 1:1 to 1:3, more particularly 1:3.

15. The method according to any of the preceding claims, wherein the enzymatic reaction is performed in the presence of Ar, particularly 10 to 90 % Ar, more particularly 20% Ar.
